(19) Europäisches Patentamt
European Patent Office
Office européen des brevets

(11) Publication number: 0 024 951
B1

(12) **EUROPEAN PATENT SPECIFICATION**

(45) Date of publication of patent specification: 07.12.83

(51) Int. Cl.[3]: C 12 P 27/00 //C12R1/645

(21) Application number: 80303091.5

(22) Date of filing: 03.09.80

# (54) Fermentation method for producing a gibberellin.

(30) Priority: 04.09.79 GB 7930585

(43) Date of publication of application: 11.03.81 Bulletin 81/10

(45) Publication of the grant of the patent: 07.12.83 Bulletin 83/49

(84) Designated Contracting States: AT BE CH DE FR GB IT LI LU NL SE

(56) References cited:
BIOCHEMICAL AND BIOPHYSICAL RESEARCH COMMUNICATIONS, vol. 91, no. 1, 14th November 1979, pages 35-40 W. RADEMACHER et al.: "Gibberellin A4 produced by sphaceloma manihoticola, the cause of the superelongation disease of cassava (manihot esculenta)

(73) Proprietor: NORSK HYDRO A/S
Bygdoy Allé 2
N-Oslo 2 (NO)

(72) Inventor: Graebbe, Jan Eiler
Gervinusstrasse 1
D-3400 Goettingen (DE)
Inventor: Rademacher, Wilhelm
Baumschulenweg 24
D-3400 Goettingen (DE)

(74) Representative: Goldin, Douglas Michael
J.A. KEMP & CO. 14, South Square Gray's Inn
London WC1R 5EU (GB)

EP 0 024 951 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 93, no. 9, 1st September 1980, page 355, no. 91991h Columbus, Ohio, U.S.A. R.S. ZEIGLER et al.: "Gibberellin A4 production by Sphaceloma manihoticola, causal agent of cassava superelongation disease"

CHEMICAL ABSTRACTS, vol. 79, no. 15, 15th October 1973, page 183, no. 89207z Columbus, Ohio, U.S.A. N. GOGALA et al.: "Growth substances in some mycorrhizal, parasitic, and saprophytic fungi"

MICROBIOLOGY ABSTRACTS, Section A, Industrial & Applied Microbiology, vol. 11, no. 3, March 1976, London, G.B. E. STRZELCZYK et al.: "Production of gibberellin-like substances by fungi isolated from mycorrhizae of pine (Pinus silvestris L.)

MICROBIOLOGY ABSTRACTS, Section A, Industrial & Applied Microbiology, vol 11, no. 3, March 1976, London, G.B. M. KAMPERT et al.: "Production of gibberellin-like substances by bacteria and fungi isolated from the roots of pine seedlings (Pinus silvestris L.)

# Fermentation method for producing a gibberellin

This invention relates to a microbiological process and, more particularly, is concerned with a new process for the production of gibberellins by fermentation.

Gibberellins are a well recognised group of tetracyclic compounds which have great value as plant growth hormones. Many gibberellins are found to occur naturally in higher plants and other gibberellins have been produced microbiologically by fermentation. Over fifty naturally occurring gibberellins have been reported in the literature and biosynthesized giberellins have also been subject to structural modification by chemical methods further increasing the number of known gibberellins. A gibberellin is regarded as a compound containing the tetracyclic ring system of ent-gibberellane shown below.

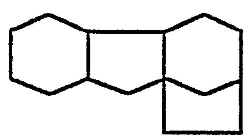

Traditionally, gibberellins are biosynthesized by the fermentation of a species of the genus *Gibberella*. High yielding strains of the ascomycete *Gibberella fujikuroi* have been developed and the fermentation of these high yielding strains provides the basis for commercial production of gibberellins.

The fermentation of *Gibberella fujikuroi* results in the formation of a mixture of gibberellins containing predominantly $GA_3$, $GA_4$ and $GA_7$. Methods have been developed for the separation of $GA_3$ from the other fermentation products but, perhaps because of the structural similarities between $GA_4$ and $GA_7$, it is more difficult to separate these two gibberellins and consequently, the commercially available product normally contains a mixture of the two gibberellins in approximately equal amounts.

Because of the potent plant growth modifying properties of the gibberellins, considerable research has been carried out since the 1950's to discover alternative microorganism that might be used as a source of gibberellins with a view to identifying organisms that would provide still higher yields of gibberellins and/or be capable of more selective biosynthesis. Since the late 1950's, a large number of microorganisms have been examined for this purpose but, prior to the present invention, there have been no reports that have been able to confirm conclusively the presence of gibberellins in the metabolites in any other microorganism other than one of the genus *Gibberella*.

We have now been able to confirm the presence of significant quantities of gibberellins in the metabolites of a microorganism which is not a member of the genus *Gibberella*.

Accordingly, the present invention provides a process for the production of a gibberellin which comprises fermenting a fungus which is gibberellin producing species of the genus *Sphaceloma* and then isolating a gibberellin from the fermentation products.

Our experimental work has concerned itself with the fermentation of the fungus responsible for the so called "super elongation" disease of the cassava crop in Colombia and which is believed to be identical with a species earlier named *Sphaceloma manihoticola*. The use of this particular microorganism in our invention is preferred. Earlier investigations into super elongation disease in cassava had not been able to identify the presence of gibberellins as being involved in the plant growth modification typical for infected plants.

*Sphaceloma manihoticola* occurs naturally in Colombia and its morphological characteristics have been reported. We have found that two separate isolates of *Sphaceloma manihoticola* have been capable of producing exceptionally high yields of gibberellins and we have deposited the two strains in the Deutsche Sammlung von Mikroorganismen (DSM), Grisebachstrasse 8, 34 Gottingen, West Germany where they have been given the deposit numbers DSM No. 1637 and DSM No. 1638.

We have surprisingly found that not only is *Sphaceloma*, a fungus morphologically distinct from the *Gibberella*, capable of producing high yields of gibberellins but also it is capable of producing a gibberellin which is substantially completely $GA_4$. This is of considerable commercial importance since it is possible to isolate the $GA_4$ from the culture by a simple solvent extraction step to give $GA_4$ substantially free from other gibberellins. It is not necessary to carry out the expensive and time consuming separations that are necessary in the existing procedures involving the fermentation of *Gibberella* and, in particular, our invention enables $GA_4$ to be produced biosynthetically free from $GA_7$.

Our fermentation is carried out under moderate fermentation conditions. We find that the fermentation temperature will normally be in the range of 15 to 30°C, preferably 27—28°C, and at a pH in the range 4 to 8, a pH of about 6.0 normally giving good results with our strains of *Sphaceloma manihoticola* on the substrates we have utilised.

We have not found the substrate composition to be unduly critical and, subject to containing adequate sources of metabolisable carbon, nitrogen and the normal trace elements, most natural or synthetic substrates are capable of supporting growth of the fungus. As a practical matter, we have been able to secure

adequate growth of the fungus on a potato dextrose medium and also on a synthetic medium based on glucose, ammonium nitrate, potassium dihydrogenphosphate and other inorganic salts to provide the necessary trace elements. We have also found it is often advantageous to use disaccharides, especially maltose, rather than monosaccharides as the carbon source.

We have found that the final yield of gibberellins produced in our fermentation process can be influenced significantly by the nitrogen content of the substrate since it is the nitrogen content that determines the duration of mycelial growth during the fermentation and controls the onset of the secondary phase of growth during which the gibberellins and other metabolites are produced.

The duration of the fermentation is governed by the criteria normally adopted in fermentation processes and, in order to maximise gibberellin yields, the fermentation should be continued for at least as long as gibberellins continue to be biosynthesized by the fungus. However, gibberellin production does fall away and no significant advantage is gained by prolonging the duration of the fermentation beyond this period.

Once the fermentation is complete, the gibberellins may be recovered from the mycelial mass by solvent extraction. Any one of the organic solvents commonly used in the processing of gibberellins may be used and we have found that esters of simple alkane carboxylic acids are most convenient. Practical considerations point to the use of alkyl esters, for example, containing 1 to 4 carbon atoms in the alkyl group, of alkanoic acids containing 2 to 5 carbon atoms and we have found that the use of ethylacetate is particularly convenient. Other organic solvents, for example, diethyl ether, may also be used for the extraction.

The solvent extract containing the gibberellins may then be subjected to purification by methods conventionally used in gibberellin chemistry and thin layer chromatography has been found by us to effect a satisfactory degree of purification of the extract.

We have used gas chromatography/mass spectrometry conclusively to identify $GA_4$ as a metabolite produced by *Sphaceloma* and, we have measured the amounts of this gibberellin by the use of gas chromatography. These measurements have shown that *Sphaceloma* is capable, under appropriate fermentation conditions, of producing $GA_4$ titres of up to 7 mg/litre.

The following Example is given to illustrate the invention.

Example

Four different nutrient media are used:

Medium 1 is a decoction of 300 g potatoes + 20 g glucose made up to a final volume of 1 l. Medium 1 was also diluted with water at the rate of 1 part of medium 1 by volume with 9 parts water by volume to form medium 2. Medium 3 is a synthetic medium of the following composition:

| | |
|---|---|
| 80 g | glucose |
| 0.48 g | $NH_4NO_3$ |
| 5.0 g | $KH_2PO_4$ |
| 1.0 g | $MgSO_4{:}7H_2O$ |
| 2.0 ml | iron stock solution* |
| 2.0 ml | micro element stock solution** |

final volume 1 l: pH 4.5

| | |
|---|---|
| *100 mg $FeSO_4{:}7H_2O$ + | **160 mg $ZnSO_4{:}7H_2O$ |
| 134 mg $Na_2EDTA$ | 15 mg $CuSO_4{:}7H_2O$ |
| solved in boiling water; | 10 mg $MnSO_4{:}7H_2O$ |
| final volume 100 ml | 10 mg $Na_2MoO_4{:}2H_2O$ |
| | ad 100 ml |

Medium 4 is identical to medium 3, but the nitrogen content is increased by utilising 2.4 g $NH_4NO_3$. Medium 5 is identical to medium 3 but the glucose is replaced by maltose and an initial pH of 6.0.

The micro-organisms used were *Sphaceloma manihoticola* No. 5 (DSM No. 1637) and No. 43 (DSM No. 1638) in the form of mycelial cultures.

100 ml portions of the nutrient medium were

introduced into a 500 ml flask and inoculated with the mycelial culture. The inoculated medium was then incubated on a rotary shaker at 21°C until fermentation was complete. This was found to take several days, and, for convenience, certain fermentations were permitted to continue for seveal weeks, although such prolonged fermentation periods are not believed to be essential.

The metabolites were then recovered from the fermentation medium after removal of mycelium by suction filtration or by centrifugation. The mycelial residue was washed with water to make the final aqueous extract 100 ml and the pH of the recovered liquid phase was adjusted to 2.7 with N-hydrochloric acid. The acidic solution was then extracted four times with 35 ml portions of ethyl acetate saturated with water. The combined ethyl acetate layers were extracted 3 times with 70 ml portions of 0.1 M $K_2HPO_4$ (saturated with ethyl acetate) which was then acidified to pH 2.7 with 5 N-hydrochloric acid, and extracted 4 times with 70 ml portions of ethylacetate saturated with water. The organic phases were combined and washed 4 times with 14 ml portions of water (whose pH was adjusted to 2.7 with hydrochloric acid). The organic phase was then concentrated under reduced pressure to remove the solvent and leave a residue of gibberellins.

The solid gibberellin residue was purified by TLC on Merck Kieselgel 60 and developed with propanol-2: 25% ammonia:water (10:1:1, by volume). The pure gibberellins have $R_f$ values: $GA_1 = 0.42$, $GA_3 = 0.47$, $GA_4 = 0.54$, $GA_7 = 0.60$, $GA_9 = 0.70$. Different zones of the chromatogram were scraped off immediately after development, homogenised in a mortar, and eluted with methanol in Pasteur pipettes. The gibberellin activity was measured with the lettuce hypocotyl bioassay (Nature 185, 255—256).

We found gibberellin activity only in the fraction having $R_f$ from 0.5 to 0.6 indicating that our gibberellin product could be $GA_4$ or $GA_7$, and that no significant quantities of $GA_3$ or $GA_1$ were present.

The extracted materials were also subjected, after methylation and trimethylsilation, to gas chromatography analysis and again, a single main component was identified having a retention time identical to that of $GA_4$ but different from that of $GA_7$.

The identity of this material as $GA_4$ was confirmed by gas chromatography/mass spectrometry using a methylated and trimethylsilylated sample of the extracted material. The gas chromatography and gas chromatography/mass spectrometry techniques failed to reveal any other gibberellins recovered from the Mycelial mass confirming the $GA_4$ selectivity of strains of *Sphaceloma manihoticola* used in our experiments.

The $GA_4$ titres obtained on our fermentations are set out in the following Table:

TABLE

| Organism *Sphaceloma manihoticola* | Medium | Duration of fermentation (days) | pH of medium after incub. | $GA_4$ content (ug/l) |
|---|---|---|---|---|
| DSM 1637 | 1 | 3 | n.d. | — |
| DSM 1638 | 1 | 3 | n.d. | — |
| DSM 1637 | 1 | 14 | n.d. | 90 |
| DSM 1638 | 2 | 11 | 7.3 | 400 |
| DSM 1637 | 3 | 64 | 4.9 | 320 |
| DSM 1638 | 3 | 64 | 4.0 | 10 |
| DSM 1638 | 4 | 76 | 6.0 | 2920 |
| DSM 1638 | 3/Ca++ | 61 | 4.0 | 30 |
| DSM 1638 | 5 (temperature of fermentation is 27°C) | 25 | 5.4 | 7320 |

Combined gas chromatography/mass spectrometry shows that in addition to $GA_4$, strains DSM 1637 and 1638 are capable of producing the following further gibberellins, the percentages indicated being percentages by weight of $GA_4$ produced in the fermentation. $GA_{13}$ (25%), $GA_{14}$ (5%) $GA_{24}$ (0.5%) $GA_9$ (0.2%) and traces of $GA_{15}$, $GA_{25}$, $GA_{36}$ and $GA_{37}$. The extremely sensitive mass fragmentation technique revealed no traces of $GA_1$, $GA_3$ or $GA_7$ in the fermentation products.

## Claims

1. A method of producing a gibberellin by fermenting a gibberellin producing species of the genus *Sphaceloma* and isolating the gibberellin from the fermentation product.

2. A method according to claim 1 wherein the species is *Sphaceloma manihoticola*.

3. A method according to claim 2 wherein the species is *Sphaceloma manihoticola* DSM 1637 or DSM 1638.

4. A method according to any one of the preceding claims wherein the fermentation is carried out at 27—28°C at an initial pH of about 6.

5. A method according to any one of the preceding claims wherein the fermentation is carried out in the presence of maltose as carbon source.

6. A method according to any one of the preceding claims wherein the gibberellin produced by fermentation is predominantly $GA_4$ and is substantially free from $GA_7$.

## Revendications

1. Procédé pour préparer une gibbereline ou acide gibberelique en faisant fermenter une espèce productrice de gibbereline du genre *Sphaceloma* et en isolant la gibbereline du produit de la fermentation.

2. Procédé selon la revendication 1, dans lequel l'espèce est *Sphaceloma manihoticola*.

3. Procédé selon la revendication 2, dans lequel l'espèce est *Sphaceloma manihoticola* DSM 1637 ou DSM 1638.

4. Procédé selon l'une quelconque des revendications précédentes, dans lequel la fermentation s'effectue à 27—28°C à un pH initial d'environ 6.

5. Procédé selon l'une quelconque des revendications précédentes, dans lequel la fermentation s'effectue en présence de maltose en tant que source de carbone.

6. Procédé selon l'une quelconque des revendications précédentes, dans lequel la gibbereline produite par fermentation est principalement $GA_4$ et ne contient pratiquement pas de $GA_7$.

## Patentansprüche

1. Verfahren zur Herstellung eines Gibberellins durch Fermentation einer Gibberellin erzeugenden Spezies der Gattung Sphaceloma und Isolierung des Gibberellins aus dem Fermentationsprodukt.

2. Verfahren nach Anspruch 1, dadurch gekennzeichnet, daß sie Spezies Sphaceloma manihoticola ist.

3. Verfahren nach Anspruch 2, dadurch gekennzeichnet, daß die Spezies Sphaceloma manihoticola DSM 1637 oder DSM 1638 ist.

4. Verfahren nach einem der voranstehenden Ansprüche, dadurch gekennzeichnet, daß die Fermentation bei 27 bis 28°C bei einem anfänglichen pH-Wert von etwa 6 durchgeführt wird.

5. Verfahren nach einem der voranstehenden Ansprüche, dadurch gekennzeichnet, daß die Fermentation in Gegenwart von Maltose als Kohlenstoffquelle durchgeführt wird.

6. Verfahren nach einem der voranstehenden Ansprüche, dadurch gekennzeichnet, daß das durch Fermentation erzeugte Gibberellin überwiegend $GA_4$ ist und im wesentlichen von $GA_7$ frei ist.